Europäisches Patentamt

European Patent Office

Office européen des brevets

(11) Publication number: **0 041 182**
**B1**

# (12) EUROPEAN PATENT SPECIFICATION

(45) Date of publication of patent specification: **21.08.85**

(51) Int. Cl.⁴: **A 61 M 5/14**

(21) Application number: **81103891.8**

(22) Date of filing: **20.05.81**

(54) **Apparatus for the parenteral administration of liquids at a constant and adjustable rate of delivery.**

(30) Priority: **21.05.80 IT 2222780**
**30.12.80 IT 2699880**

(43) Date of publication of application:
**09.12.81 Bulletin 81/49**

(45) Publication of the grant of the patent:
**21.08.85 Bulletin 85/34**

(84) Designated Contracting States:
**AT BE CH DE FR GB LI NL SE**

(56) References cited:
**FR-A- 757 231**
**FR-A-2 434 626**
**US-A-3 931 818**

(73) Proprietor: **SIS-TER S.p.A.**
**Via Crema, 14**
**I-26020 Palazzo Pignano (Cremona) (IT)**

(72) Inventor: **di Salvo, Francesco**
**Via per Roncate 10**
**I-22100 Como (IT)**

(74) Representative: **Von Bezold, Dieter, Dr.**
**Patentanwälte Dr.Dieter v. Bezold et al**
**Dipl.-Ing. Peter Schütz Dipl.-Ing. Wolfgang**
**Heusler Postfach 860260 Maria-Theresia-Strasse**
**22**
**D-8000 München (DE)**

# Description

The present invention relates to an apparatus for parenterally administering liquid, comprising a sealed dripping chamber having a liquid inlet at its top and a liquid outlet at its bottom, said inlet being adapted for connection to a vessel containing the liquid to be administered, a flow rate stabilizing device having a chamber with a liquid inlet passage connected to said liquid outlet of said dripping chamber, and with a liquid outlet passage opening through the body of the flow stabilizing device upwardly into said chamber and being adapted for connection to a cannula, and a movable flow control element actuated by the liquid in said stabilizing device chamber to control the flow of liquid from said chamber to said outlet passage, a pressure equalizing conduit connecting said dripping chamber and said flow rate stabilizing device chamber at points above the level of the liquid within these chambers, and means comprising a scale for adjusting the height of the stabilizing device with respect to said dripping chamber.

A known apparatus of this type described in US—A—3,391,818 (Goldowsky) comprises a movable flow control element in the form of a float which actuates a needle-type valve.

Other known apparatus for the parenteral administration of liquid with a constant adjustable rate comprise one or more capillary tubes as flow control means (US—A—3,878,879; US—A—3,298,367).

The above mentioned known apparatus are either complicated and delicate to operate or suffer from poor flow rate control.

The invention as claimed is intended to remedy these drawbacks. It solves the problem to provide an apparatus for parenterally administering liquid which is simple in construction and nevertheless allows a close control of the flow rate of the liquid administered.

The advantages offered by the invention are mainly that the present apparatus operates in a purely hydro-pneumatic manner and is insensitive against tilting which would tend to impair the operation of a float. The apparatus according to the invention is specifically useful in connection with liquid containers having flexible walls.

In a preferred embodiment of the present apparatus, the pressure equalizing conduit is of capillary type.

An inlet passage of capillary size of said stabilizing device is another preferred feature of the present apparatus.

In a preferred embodiment of the present apparatus, the body of the flow stabilizing device has an upper face defining the stabilizing device chamber and formed with an annular outer projection and a central projection, said flow stabilizing device further comprising a pressure element clamping the membrane along said annular projection and leaving it free to rest on the central projection, and said outlet passage communicates with said chamber through a hole in the central zone of said central projection.

A preferred embodiment of the invention will now be described with reference to the drawings, in which

Figure 1 is a perspective view of an apparatus according to a preferred embodiment of the present invention;

Figure 2 is a more detailed front view on an enlarged scale of a dripping chamber of a liquid flow rate adjusting and stabilizing device of the apparatus shown in Fig. 1;

Figure 3 illustrates a portion of a graduated rod and a sliding member for supporting the flow rate adjusting and stabilizing device of the apparatus shown in Fig. 1, and

Figure 4 shows an axial cross-section, on enlarged scale, of the flow rate adjusting and stabilizing device of the apparatus shown in Fig. 1.

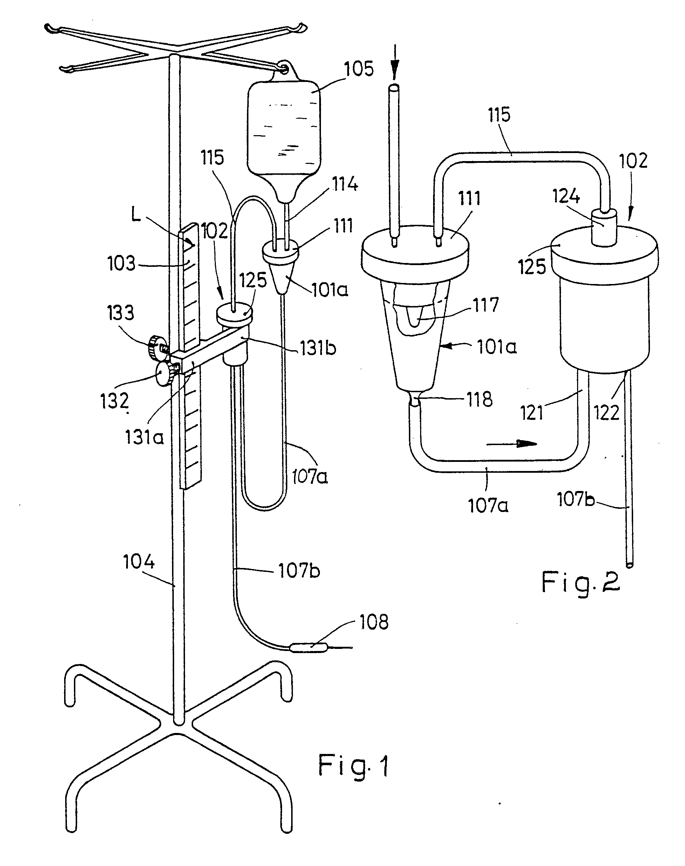

Referring to Figures 1 to 4, the apparatus shown, in order to hold the flow rate constant, comprises a liquid flow rate adjusting-stabilizing device 102, provided, in the inside thereof, with a deformable membrane M, effective to communicate one with another the two channel-like passages 121 and 122, respectively, for the inlet and the outlet of the liquid, through the body of the device 102, in such a way as to hold the flow constant. More specifically, the channel-like passage 122, which has a diameter larger than that of the passage 121, is connected or coupled, by means of a flexible tube 107b, to a cannula or needle 108 for administering parenterally the liquid.

The inlet passage 121, on the other hand, is connected, through another flexible tube branch 107a, to a dripping chamber 101a. This latter is provided, at the top thereof, with sealed covering means consisting of a two way ring nut 111, which is passed through by two through tubes, one thereof, indicated at 114 in Figures 1 and 2, providing connection with a vessel 105 of the liquid to be adminstered, said vessel being, as it should be apparent, either a flexible bag or a rigid vessel, such as a glass or rigid plastics material bottle.

The liquid enters the dripping chamber 101a through a perforated end member 117, having preferably, but not necessarily, a diameter within a range from 0,3 to 3 mm. In particular the cup or dripping chamber 101a can be made of a flexible or rigid plastics material, and it, at the bottom thereof, is provided with an end member 118 to be coupled to the tube 107a for introducing the liquid into the flow rate adjusting and stabilizing device 102, as above stated.

The second tube passing through the ring nut 111, indicated at 115 in Figures 1 and 2, consists of a flexible tube connecting with an end member 124 of means for sealingly covering the flow adjusting device 102, and consisting of a cover 125. Thus, the inside of the dripping chamber 101a and the inside of the liquid flow rate adjusting and stabilizing device 102 are communicated one with the other, thereby providing one of the main characteristics of the instant apparatus, consisting of the fact that the pressure in the inside of the dripping chamber 101a is transmitted, substantially unaltered, onto the membrane of the flow rate adjusting and stabilizing device. To this end, the duct 115 will have a greatly reduced inner diameter of such a value effective to transmit nearly instantaneously to the flow rate adjusting and stabilizing device 102 the pressure variations occurring in the inside of said dripping chamber 101a.

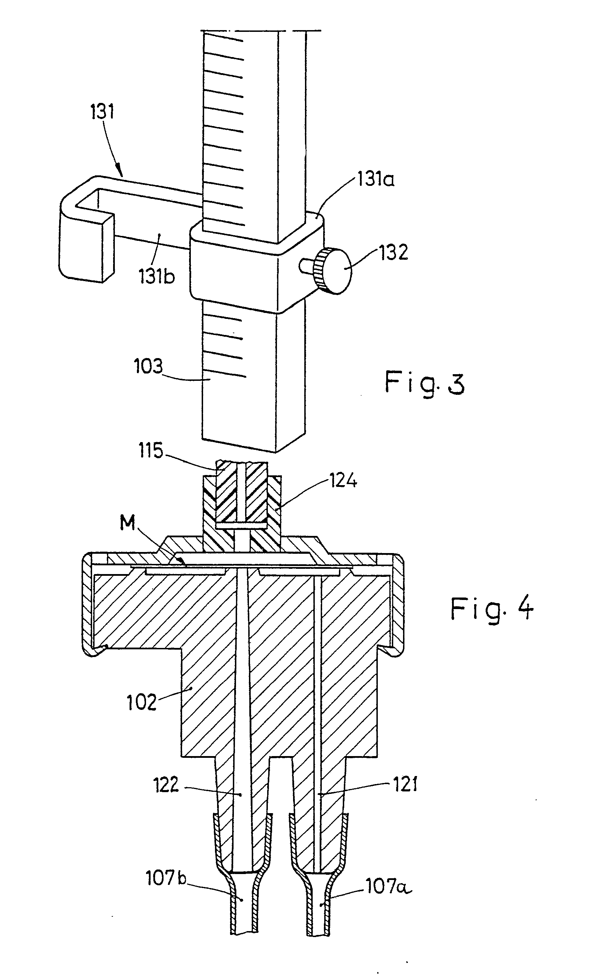

Referring now to Figures 1 and 3, on a conventional stationary supporting member 104, to one arm thereof there is fixed the vessel of the liquid to be administered, consisting of a deformable or flexible bag 105 in the illustrated embodiment, there is fixed a graduated rod 103, provided with a sliding member or slider 131. This latter comprises a ring portion 131a, slidably supporting said graduated rod 103, and a shaped projecting portion 131b, providing a seat for housing and supporting the device 102. The locking of the sliding member 131 on said graduated rod 103 is carried out by using a clamp member 132 and, in turn, the graduated rod 103 can be set at the desired level or height on the supporting member 104 by means of a further clamp member 133, schematically shown in Figure 1.

The operation of the above described embodiment of the inventive apparatus, wherein a flexible wall vessel is used, is the following.

Firstly the apparatus is calibrated, upon having locked the graduated rod or scale 103 on said supporting member 104 at the desired height, depending on the operator height. This calibration stage is carried out by locating at the same height the level of the liquid in the dripping chamber 101a, the liquid flow rate adjusting and stabilizing device 102 and a reference mark of the graduated rod or scale 103, related to the calibration, and indicated at L in Figure 1. In this position, and supposing that a shut-off valve in the duct 114 is in the open position thereof, then there will be no flow of liquid through the flow rate adjusting and stabilizing device 102, since the pressures exerted on the two faces of the membrane are equal and, accordingly, said membrane prevents the liquid from flowing from one duct to the other. In fact this pressure value depends on the level of the liquid in the vessel 105 which, since it does not supply any liquid at this time, is at a fixed height.

In order to obtain the desired flow rate, the sliding member 131 has to be displaced to a lower position on the graduated rod 103, at the mark thereof indicating said desired flow rate, and then, said sliding member is locked at this position. Because of the level difference existing between the liquid level in the dripping chamber 101a and the membrane, a pressure difference will exist between the two sides of the membrane, which will allow the liquid to flow through the passages 121 and 122. More specifically the pressure on the top of said membrane, which pressure is transmitted to the flow rate adjusting and stabilizing device 102 through the duct 115, is that present in the upper portion of the dripping chamber 101a and depends on the liquid level in the vessel 105. At the start of the delivery this pressure is equal to the calibration pressure.

On the other hand, the pressure acting on the lower side of the membrane corresponds to the sum of the preceding value and that deriving from the level difference between the liquid in the dripping chamber 101a and the membrane. The difference between these values, which difference will depend on the level at which said flow rate adjusting and stabilizing device 102 is set, is the overpressure value causing said membrane to deform and the liquid to flow.

During the dispensing of the liquid, as the liquid level in the vessel 105 decreases, the pressure in the dripping chamber 101a, and thus the pressure exerted on the top of said membrane through the duct 115 will also decrease. On the other hand, the flow rate does not decrease since this pressure decrease is compensated for by a corresponding or like pressure decrease on the membrane lower face, which pressure is transmitted by said fluid through the duct 107a. Each pressure variation in the dripping chamber 101a, in particular at the liquid inlet zone in said dripping chamber, is therefore transmitted simultaneously onto the two faces of the membrane which is biassed only to open the liquid passage port by the pressure value determined by the level difference existing between the membrane and the dripping chamber.

Thus the invention provides essentially a feedback circuit effective to transmit the same pressure values, which decrease in time, on the two faces of the membrane: accordingly they do not alter the constant pressure value as determined or set by the different height of the liquid flow rate adjusting and stabilizing device and of the dripping chamber 101a.

It should be apparent that, at the end of the liquid dispensing operation, since the disclosed system is a closed one, the air will be prevented from entering the dripping chamber 101a, thereby it will not enter the patient's vein.

From the above description it should be noted that the inventive apparatus described with reference to Figures 1 to 4 can also be used, without any variations, with rigid wall liquid vessels, such as glass bottles. In this case in the vessel a passage is provided for the inlet of air, so that the pressure will not decrease as the level of the liquid in the vessel decreases, but, on the contrary, will be held constant and equal to the atmospheric pressure. This pressure will be transmitted, in the same manner as described above, onto the two faces of the membrane, and the parameter determining the flow rate will be, also in this case, the level difference between the flow rate adjusting and stabilizing device and the dripping chamber 101a. Also in this case the calibration operations are carried out as indicated.

Self-evident is furthermore the advantage of providing a precisely constant flow rate for long periods of time, by means of a simple calibrating procedure which does not involve test administration at flow rate values different from the predetermined one, which are potentially dangerous for the patient. Finally it should be also noted that the flow rate can be easily adjusted by simply shifting the slider 131.

Modifications and variations can be made to the illustrated embodiments of the apparatus according to the invention without departing from the scope thereof as defined by the appended claims.

### Claims

1. An apparatus for parenterally administering liquid comprising

a sealed dripping chamber (101a) having a liquid inlet at its top and a liquid outlet at its bottom, said inlet being adapted for connection to a vessel (105) containing the liquid to be administered,

a flow rate stabilizing device (102) having a chamber with a liquid inlet passage (121) connected to said liquid outlet of said dripping chamber (102), and with a liquid outlet passage (122) opening through the body of the flow stabilizing device upwardly into said chamber and being adapted for connection to a cannula, and a movable flow control element (M) actuated by the liquid in said stabilizing device chamber to control the flow of liquid from said chamber to said outlet passage,

a pressure equalizing conduit (115) connecting said dripping chamber and said flow rate stabilizing device chamber at points above the level of liquid within these chambers, and

means (103, 131, 132, 133) comprising a scale (103) for adjusting the height of the stabilizing device (102) with respect to said dripping chamber (101a), characterized in that

said inlet passage (121), which also opens upwardly into said flow stabilizing device chamber has a smaller diameter than said outlet passage (122), and that said movable flow control element is a membrane (M) having a first face in communication with said pressure equalizing conduit (115) and a second face in communication with the openings of said passages (121, 122) and adapted to seal the opening of said outlet passage (122) in response to the absence of a difference of the pressures acting on its faces.

2. The apparatus as claimed in claim 1 wherein said pressure equalizing conduit (115) is of capillary type.

3. The apparatus as claimed in claims 1 or 2, wherein said inlet passage (121) of said stabilizing device (102) of capillary size.

4. The apparatus as claimed in any of claims 1, 2 or 3, wherein the body of said flow stabilizing device (102) has an upper face defining said chamber and formed with an annular outer projection and a central projection,

said flow stabilizing device further comprising a presser element clamping said membrane (M) along said annular projection and leaving it free to rest on said central projection, and

said outlet passage communicates with said chamber through a hole in the central zone of said central projection.

### Patentansprüche

1. Gerät für die parenterale Verabreichung einer Flüssigkeit mit

einer abgeschlossenen Tropfkammer (101a), die oben eine Flüssigkeitseinlaß, der an ein die zur vera breichende Flüssigkeit enthaltendes Gefäß (101) anschließbar ist, und unten einen Flüssigkeitsauslaß hat,

einer Vorrichtung (102) zur Stabilisierung der Strömungsrate, die eine Kammer mit einem Flüssigkeitseinlaßkanal (121), der mit der Tropfkammer (102) verbunden ist, und mit einem Flüssigkeitsauslaßkanal (122), der durch den Körper der Vorrichtung zur Stabilisierung der Stromungsrate nach oben in der Kammer mündet und an eine Kanüle anschließbar ist, und ein bewegliches Strömungssteuerelement (M) enthält, welches durch die Flüssigkeit in der Kammer in der Stabilisierungsvorrichtung betätigt ist und die Strömung der Flüssigkeit aus der Kammer zum Auslaßkanal steuert, einer Druckausgleichsleitung (115), die mit der Tropfkammer und der Kammer der Vorrichtung zur Stabilisierung der Strömungsrate an Punkten oberhalb des Flüssigkeitsspiegels in diesen Kammern verbunden ist, und einer Vorrichtung (103, 131, 132, 133), die eine Skala (103) zur Einstellung der Höhe der Stabilisierungsvorrichtung (102) bezüglich der Tropfkammer (101) enthält, dadurch gekennzeichnet, daß der Einlaßkanal (121), der ebenfalls nach oben in der Kammer der Strömungsstabilisierungsvorrichtung mündet, einem kleineren Durchmesser als der Auslaßkanal (122) hat und daß das bewegliche Strömungssteuerelement eine Membrane (M) ist, deren eine Seite mit der Druckausgleichsleitung (115) deren andere Seite mit den Mündungen der genannten Kanäle (121, 122) in Verbindung stehen und die die Mündung des Auslaßkanals als Reaktion auf das Fehlen einer auf ihre Seiten wirkenden Druckdifferenz zu verschließen gestattet.

2. Gerät nach Anspruch 1, dadurch gekennzeichnet, daß die Druckausgleichsleitung (115) vom Kapillartyp ist.

3. Gerät nach Anspruch 1 oder 2, dadurch gekennzeichnet, daß der Einlaßkanal (121) der Stabilisierungsvorrichtung (102) kapillare Abmessung hat.

4. Gerät nach einem der Ansprüche 1, 2 oder 3, dadurch gekennzeichnet, daß der Körper der Strömungsstabilisierungsvorrichtung (102) eine obere Seite aufweist, die die Kammer begrenzt und einen ringförmigen äußeren Vorsprung sowie einen mittleren Vorsprung bildet,

daß die Strömungsstabilisierungsvorrichtung außendem ein Druckelement enthält, das die Membran (M) längs des ringförmigen Vorsprunges festklemmt und sie auf dem mittleren Vorsprung frei ruhen läßt und

daß der Auslaßkanal mit der Kammer durch ein Loch in der mittleren Zone des mittleren Vorsprunges in Verbindung steht.

## Revendications

1. Appareil d'administration parentérale de liquide comprenant

une chambre d'écoulement goutte à goutte étanche (101a) comportant une entrée de liquide à sa partie supérieure et une sortie de liquide à sa partie inférieure, ladite entrée étant adaptée pour être reliée à un récipient (105) contenant le liquide à administrer,

un dispositif stabilisateur de débit (102) comportant une chambre pourvue d'un passage d'entrée du liquide (121) reliée à ladite sortie de liquide de la chambre d'écoulement goutte à goutte (101a), ainsi que d'un passage de sortie du liquide (122) débouchant vers le haut, à travers le corps du dispositif stabilisateur de débit dans ladite chambre et adapté pour être relié à une canule, et un élément mobile de réglage de débit (M), actionné par le liquide se trouvant dans ladite chambre du dispositif stabilisateur pour régler le débit de liquide de ladite chambre vers ledit passage de sortie,

un conduit d'égalisation de pression (115) reliant la chambre d'écoulement goutte à goutte et la chambre du dispositif stabilisateur de débit en des points situés au-dessus du niveau de liquide à l'intérieur de ces chambres, et

des moyens (103, 131, 132, 133) comprenant un échelle graduée (103) pour ajuster la hauteur du dispositif stabilisateur (102) par rapport à la chambre d'écoulement goutte à goutte (101a),

caractérisé en ce que le passage d'entrée (121), qui débouche également vers le haut dans la chambre du dispositif stabilisateur de débit, présente un diamètre plus petit que celui du passage de sortie (122), et en ce que l'élément mobile de réglage de débit est une membrane (M) comportant une première face en communication avec le conduit d'égalisation de pression (115) et une seconde face en communication avec les ouvertures des passages (121, 122) et adaptée pour obturer l'ouverture dudit passage de sortie (122) en réaction à l'absence de différence entre les pressions agissant sur ses faces.

2. Appareil selon la revendication 1, dans lequel le conduit d'égalisation de pressions (115) est de type capillaire.

3. Appareil selon la revendication 1 ou 2, dans lequel le passage (121) du dispositif stabilisateur (102) est de dimension capillaire.

4. Appareil selon l'une des revendications 1, 2 ou 3, dans lequel le corps du dispositif stabilisateur de débit (102) comporte une face supérieure délimitant ladite chambre et formée par une saillie extérieure annulaire et une saillie centrale, ledit dispositif stabilisateur de débit comprenant en outre un élément presseur serrant la membrane (M) contre la saillie annulaire et la laissant libre de reposer sur ladite saillie centrale, et ledit passage de sortie communiquant avec ladite chambre par un trou formé dans la zone centrale de ladite saillie centrale.

Fig·1

Fig.2

131

131a

131b

132

103

Fig. 3

115

124

M

Fig. 4

102

122

121

107b

107a